# EUROPEAN PATENT SPECIFICATION

(11) **EP 2 588 867 B1**
(45) Date of publication and mention of the grant of the patent: **25.01.2017**
(21) Application number: 11730394.1
(22) Date of filing: 29.03.2011
(51) Int. Cl.: G01N 33/82

(54) **DETECTION AND QUANTIFICATION OF VITAMIN D METABOLITES USING AN ALKYLAMINE TO FORM STABLE ADDUCTS FOR MASS SPECTROMETRIC ANALYSIS**
DETEKTION UND QUANTIFIZIERUNG VON VITAMIN D-METABOLITEN UNTER VERWENDUNG EINES ALKYLAMINS UM STABILE ADDUKTE FÜR DIE MASSENSPEKTROMETRISCHE ANALYSE ZU BILDEN
DÉTECTION ET QUANTIFICATION DES MÉTABOLITES DE LA VITAMINE D EN UTILISANT UN ALKYLAMINE POUR FORMER DES ADDUITS STABLES POUR L'ANALYSE SPECTROMÉTRIE DE MASSE

(30) Priority: 16.07.2010 US 365030 P; 30.06.2010 GB 201011119
(43) Date of publication of application: 08.05.2013
(73) Proprietor: Micromass UK Limited, Wilmslow SK9 4AX (GB)
(72) Inventor: CALTON, Lisa J., Stockport SK7 4JA (GB); MOLLOY, Billy J., Stockport SK3 8SL (GB)
(74) Representative: Harding, Andrew Philip
(86) International application number: PCT/GB2011/050628
(87) International publication number: WO 2012/001378

(56) References cited:
- TATSUYA HIGASHI ET AL: "Liquid chromatographyâ tandem mass spectrometric method for the determination of salivary 25-hydroxyvitamin D3: a noninvasive tool for the assessment of vitamin D status", ANALYTICAL AND BIOANALYTICAL CHEMISTRY, SPRINGER, BERLIN, DE, vol. 391, no. 1, 18 December 2007 (2007-12-18), pages 229-238, XP019621231, ISSN: 1618-2650
- TESHIMA K ET AL: "Analytical method for ubiquinone-9 and ubiquinone-10 in rat tissues by liquid chromatography/turbo ion spray tandem mass spectrometry with 1-alkylamine as an additive to the mobile phase", ANALYTICAL BIOCHEMISTRY, ACADEMIC PRESS INC, NEW YORK, vol. 338, no. 1, 1 March 2005 (2005-03-01) , pages 12-19, XP004747511, ISSN: 0003-2697, DOI: DOI:10.1016/J.AB.2004.12.007
- Lisa J Calton ET AL: "THE ANALYSIS OF 25-HYDROXYVITAMIN D IN SERUM USING UPLC/MS/MS", , 1 October 2008 (2008-10-01), pages 1-4, XP55001300, [retrieved on 2011-06-23]
- LENSMEYER GARY L ET AL: "HPLC method for 25-hydroxyvitamin D measurement: Comparison with contemporary assays", CLINICAL CHEMISTRY, vol. 52, no. 6, June 2006 (2006-06), pages 1120-1126, XP002654800, ISSN: 0009-9147
- HIGASHI TATSUYA ET AL: "Derivatization of neutral steroids to enhance their detection characteristics in liquid chromatography-mass spectrometry.", ANALYTICAL AND BIOANALYTICAL CHEMISTRY FEB 2004 LNKD- PUBMED:14598004, vol. 378, no. 4, February 2004 (2004-02), pages 875-882, XP002654801, ISSN: 1618-2642
- SHIMADA K ET AL: "Gas chromatography and high-performance liquid chromatography of natural steroids", JOURNAL OF CHROMATOGRAPHY, ELSEVIER SCIENCE PUBLISHERS B.V, NL, vol. 935, no. 1-2, 23 November 2001 (2001-11-23), pages 141-172, XP004322069, ISSN: 0021-9673, DOI: DOI:10.1016/S0021-9673(01)00943-8
- GAO ET AL: "Sensitivity enhancement in liquid chromatography/atmospheric pressure ionization mass spectrometry using derivatization and mobile phase additives", JOURNAL OF CHROMATOGRAPHY B: BIOMEDICAL SCIENCES & APPLICATIONS, ELSEVIER, AMSTERDAM, NL, vol. 825, no. 2, 25 October 2005 (2005-10-25), pages 98-110, XP005102433, ISSN: 1570-0232, DOI: DOI:10.1016/J.JCHROMB.2005.04.021

## Description

The present invention relates to detection and quantification of vitamin D metabolites particularly to a method for enhancing the signal detected when mono, di or tri-hydroxy metabolites of vitamins D3 and D2 are analysed by mass spectrometry.

Vitamin D is a generic designation for a group of fat-soluble structurally similar sterols. Vitamin D compounds are derived from dietary ergocalciferol (from plants, vitamin D2) or cholecalciferol (from animals, vitamin D3), or by conversion of 7-dihydrocholesterol to vitamin D3 in the skin upon UV-exposure. Vitamin D2 and D3 are subsequently 25-hydroxylated in the liver to form 25-hydroxyvitamin D2 (25OHD2) and 25-hydroxyvitamin D3 (25OHD3). 25OHD2 and 25OHD3 represent the main body reservoir and transport form of vitamin D. They are stored in adipose tissue or are tightly bound by a transport protein while in circulation, and are subsequently hydroxylated to the corresponding 1,25-dihydroxy forms in the kidney. 1,25-dihydroxyvitamin D2 (DHVD2) and 1,25-dihydroxyvitamin D3 (DHVD3) are potent calciotropic hormones involved in the regulation of both calcium and phosphate metabolism, and are inhibitors of parathyroid hormone (PTH).

Vitamin D laboratory testing has increased significantly during the last decade because of an increasing awareness that vitamin D deficiency is very common and can increase fracture and, possibly, cancer risk. Measurement of total 25-hydroxyvitamin D (25OHD; sum of 25OHD2 and 25OHD3) is the preferred test for assessing vitamin D status, because it has a long serum half-life and its concentration is considered to be in equilibrium with vitamin D body stores.

Unfortunately, there are substantial discrepancies between test results obtained with different 25OHD assays. Most 25-OHD assays are competitive immunoassays or competitive assays based on vitamin D binding proteins. For such assays, 25OHD is a difficult analyte because of its hydrophobicity and relatively low serum concentrations. This often necessitates sample extraction and concentration before analysis, potentially increasing assay variability. Furthermore, equal detection of 25OHD2 and 25OHD3 represents a challenge, in particular for assays based on vitamin D binding protein, because binding proteins from many species show higher affinity for 250HD3 than for 250HD2. As a consequence of all these factors, only 50- 60% of the approximately 100 laboratories that participate in the international quality assessment scheme for vitamin D metabolites (DEQAS), meet performance criteria consistently, and the results obtained for the same sample can differ up to 2- to 4-fold, sometimes even for the same assay, when performed in different laboratories.

It is known from WO 2010/019566 that methods can be used to measure the levels of DHVD2, DHVD3, or both (total DHVD) in a sample. For example, DHVD2 and DHVD3 can be selectively and sensitively detected and quantitated using methods employing affinity purification, analyte derivatization, and mass spectrometric (MS) techniques. WO 2010/019566 discloses that the combination of the affinity purification and analyte derivatization steps eliminates sample interferences, provides increased sensitivities, and provides more accurate results than methods that employ only analyte derivatization and concludes that the disclosed methods can facilitate reliable quantification of both DHVD2 and DHVD3 to 5 pg/mL or lower. The materials and methods are said to be useful to aid in the diagnosis of vitamin D deficiencies or hypervitaminosis D, to monitor vitamin D replacement therapies, and to aid in the diagnosis of various disorders, e.g., hypercalcemia, chronic renal failure, hypoparathyroidism, sarcoidosis, granulomatous diseases, malignancies, primary hyperparathyroidism, and physiologic hyperarathyroidism.

Higashi and coworkers (Anal Bioanal Chem. 2008. 391:229-238) describe a sensitive liquid chromatography-electrospray ionization-tandem mass spectrometric (LC-ESI-MS/MS) method for the determination of the PTAD derivative of 25-hydroxyvitamin D3 in human saliva.

According to the present invention there is provided a method of determining the amount of an analyte in a sample wherein the analyte comprises one or more vitamin D metabolites;
comprising the steps of:
   contacting the analyte with an alkylamine to form an alkylamine adduct, wherein the amine is a monoamine or diamine and wherein the alkylamine is a C₄-C₁₂ alkylamine;
   subjecting the adduct to a MS technique to determine the amount of the adduct; and
   calculating the amount of the analyte equivalent to the amount of the adduct, wherein the method does not include a derivatisation step.

The vitamin D metabolite preferably comprises one or more compounds selected from the group consisting of:-
1-alpha-hydroxyvitamin D2, 1-alpha-hydroxy vitamin D3, 25 -hydroxy vitamin D2, 25-hydroxyvitamin D3, 1,25-dihydroxyvitamin D2, 1,25-dihydroxyvitamin D3, (R) 24, 25 - dihydroxyvitamin D2, (R)24,25 dihydroxyvitamin D3,(S) 24,25-dihydroxyvitamin D2, (S)24,25 dihydroxyvitamin D3, 1,24,25-trihydroxyvitamin D2, and 1,24,25-trihydroxyvitamin D3.

Mixtures of two or more of these metabolites may be detected and quantified by the method of this invention.

Use of a monoamine is preferred, especially a straight chain saturated alkylamine. The alkylamine comprises C₄-C₁₂, preferably C₄-C₈ more preferably C₆-C₈ alkylamines. Use of hexylamine or octylamine are especially preferred.

Diamines which may be employed have the formula H₂N(CH₂)ₙNH₂ wherein n is an integer from 2-12, preferably an even integer from 2-12.

The alkylamine may be added to the liquid sample followed by the step of UPLC/HPLC separation of the sample.

Alternatively the alkylamine may be added to the liquid chromatography flow prior to MS detection.

In a further possibility the alkylamine may added to the sample pre or post extraction.

The sample may be a dried extracted sample. In this case the method may include the step of adding the dried extracted sample to a solution containing the alkylamine.

The MS technique preferably comprises a tandem (MS/MS) technique. Use of an LC- MS/MS technique is especially preferred. The MS technique may comprise a triple quadrapole technique wherein Multiple Reaction Monitoring (MRM) in positive ion mode is employed. The LC-MS/MS technique may comprise a Ql scan tuned to detect a precursor that corresponds to the analyte-amine adduct. For example for detection of 1,25-dihydroxyvitamin D3. The Ql peak may comprise MRM 518.6/102.1 when hexylamine is used and 546.6/130.2 when octylamine is used to form the adduct.

The invention is further described by means of example but not in any limitative sense with reference to the accompanying drawings of which;
Figure 1 is a full scan spectrum of the chromatographic peak for 1,25-dihydroxyvitamin D3 when octylamine is introduced post-column;
Figure 2 is the MRM spectrum of the hexylamine adduct of 1,25-dihydroxyvitamin D3 at a collision energy of 10eV;
Figure 3 is a chromatogram demonstrating assay of 1,25-dihydroxyvitamin D3, and
Figure 4 is a graph showing linearity for the formation of the octylamine adduct of 1,25-dihydroxyvitamin D3.

Liquid chromatography was carried out using two sets of parameters.

### Method 1

Mobile phase A: Water with 0.05% formic acid
Mobile phase B: MeOH with 0.05% formic acid
Weak wash solvent: Water
Strong wash solvent :ACN
Column: ACQUITY BEH 2.1 x50mm C18
Column temp: 40°C
Injection Vol: 20 µL (Full Loop)
Flow Rate: 0.35ml/min

| Gradient: | | Run time: 4.6 mins | |
|---|---|---|---|
| Time | %A | %B | curve |
| 0 | 40 | 60 | 1 |
| 4 | 2 | 98 | 6 |
| 4.1 | 2 | 98 | 6 |
| 4.2 | 40 | 60 | 6 |
| 4.5 | 40 | 60 | 6 |

Various concentrations (0.1-2mM of octylamine in 60% methanol (aqueous) were infused, post-column, using the fluidics system to produce adducts.

### Method 2

Mobile phase A: Water with 0.1% formic acid and 0.1mM hexylamine
Mobile phase B: MeOH with 0.1% formic acid and 0.1mM hexylamine
Weak wash solvent - Mobile Phase A
Strong wash solvent: Mobile Phase B
Column: ACQUITY UPLC BEH Shield RP18 2.1x50mm
Column temp: 75°C
Injection Vol: 50 µL (Full Loop)
Flow Rate: 0.5mL/min

| Gradient: | | Run time: 3 mins | |
|---|---|---|---|
| Time | %A | %B | curve |
| 0 | 50 | 50 | 1 |
| 0.2 | 50 | 50 | 6 |
| 2.0 | 0 | 100 | 6 |
| 2.8 | 50 | 50 | 6 |

Mass spectrometry was carried out using the following conditions:
The instrument was tuned for unit resolution for MS1 (0.7Da FWHM) and the resolution for MS2 (0.8-0.9Da FWHM).

| MS Conditions | |
|---|---|
| Polarity | ES+ |
| Capillary (kV) | 3.0 |
| Cone (V) | 20.0 |
| Extractor (V) | 3.00 |
| RF (V) | 0.1 |
| Source Temperature (°C) | 120 |
| Desolvation Temperature (°C) | 350 |
| Cone Gas Flow (L/Hr) | 20 |
| Desolvation Gas Flow (L/Hr) | 1000 |
| Collision Gas Flow (ml/min) | 0.15 |

**MRM Transitions**

| Compound | MRM | Dwell (secs) | Cone Voltage (V) | CollisionEnergy (eV) |
|---|---|---|---|---|
| [1,25diOHD3+HA]⁺ | 518.6 > 102.1 | 0.08 | 20 | 28 |
| [1,25diOHD3+OA]⁺ | 546.6 > 130.2 | 0.08 | 20 | 10 |

### Samples were prepared as follows:

1,25diOHD3, 1,25diOHD2, (R)24,25diOHD3 and (S)24,25diOHD3 were purchased from Sigma-Aldrich and were each dissolved in ethanol to produce 2mg/mL, 2mg/mL, 0.5mg/mL and 1mg/mL standards respectively. These solutions were kept in the freezer until required. Solutions were prepared at various concentrations from the primary stocks by diluting with 60% methanol (aqueous). Hexylamine (MW 101.2) and octylamine (MW 129.2) were purchased from Sigma-Aldrich. Solutions were prepared at various concentrations by dilution with 60% aqueous methanol.

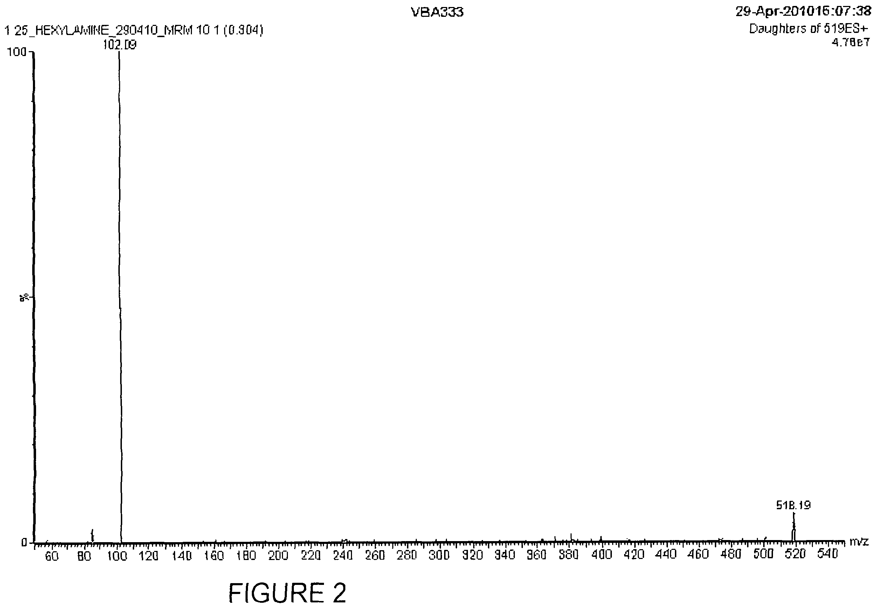

Full scan data was acquired simultaneously with the MRM date using Method 1, infusing oxylamine post-column. The spectrum in Figure 2 was extracted from the full scan data at the retention time of 1,25diOHD3.

A full scan spectrum of chromatographic peak for 1 µg/mL solvent standard injection of 1,25di OHD3 when octylamine is introduced post-column. [M+OA]⁺ (m/z +546.6) was observed to be the most abundant ion.

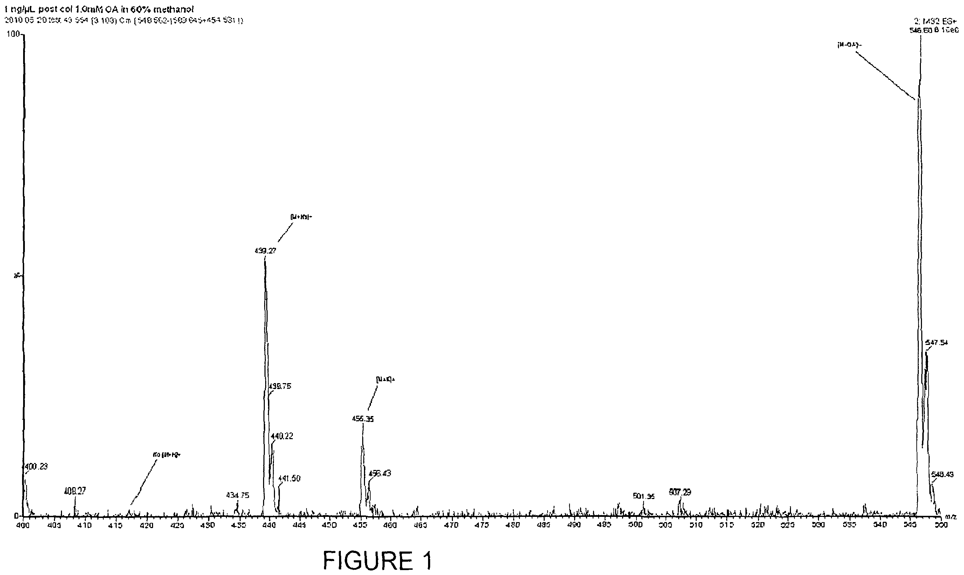

1,25diOHD has been found to form a sodium adduct [M+NA]⁺. This causes lowering of the sensitivity for the assay significantly due to a difficulty in fragmenting this species to form product ions in the collision cell of the mass spectrometer. Addition of octylamine (OA) resulted in the dominant precursor ion being the [M+OA]⁺ adduct. Protonated species were not observed. By adjustment of the concentration of alkylamine the levels of sodium adducts were reduced. The alkylamine adduct was observed to readily dissociate in the collision cell of the mass spectrometer to form a protonated alkylamine ion as the dominant or only product ion (m/z=102). This is shown in Figure 1. An enhancement in sensitivity in relation to other analytical methods was therefore obtained.

It is an advantage of this invention that the concentration of alkylamine required to form an adduct may be very low. A concentration of 0.1mM of hexylamine added to the mobile phase resulted in the dominant precursor ion being the hexylamine adduct with protonated species not being observed.

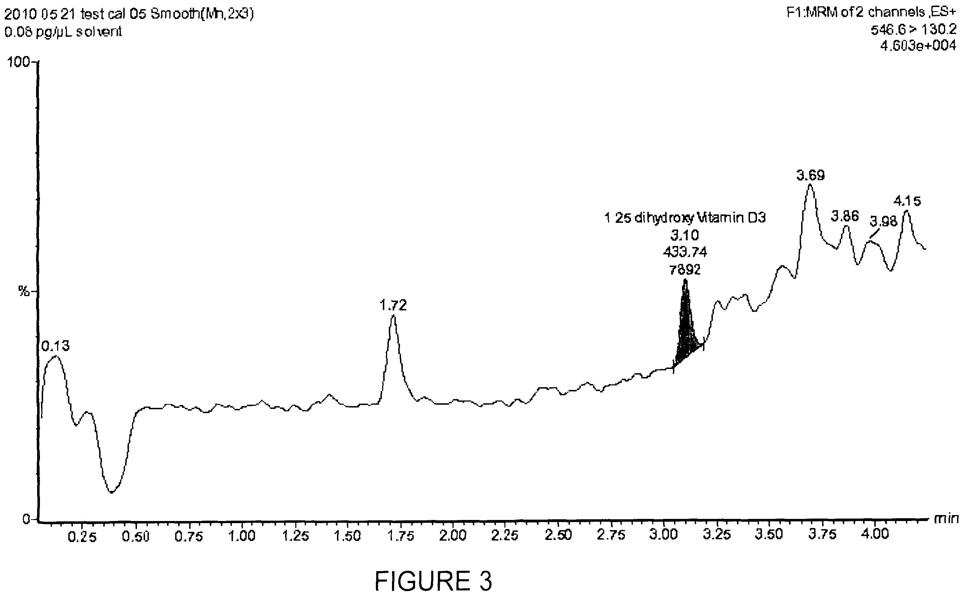

Figure 3 shows a chromatogram demonstrating the possibility of reaching the low limits of detection required for this assay.

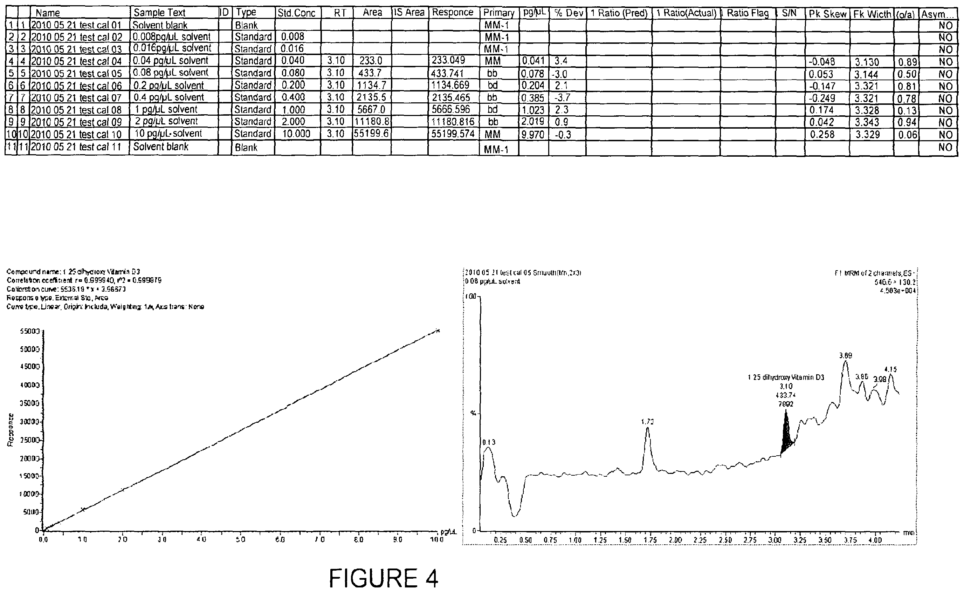

Figure 4 shows the alkylamine adduct formation for 1,25diOHD3 to be linear over the concentration range 40-10,000pg/mL.

## Claims

1. A method of determining the amount of an analyte in a sample wherein the analyte comprises one or more vitamin D metabolites;
comprising the steps of:
contacting the analyte with an alkylamine to form an alkylamine adduct, wherein the amine is a monoamine or diamine and wherein the alkylamine is a C₄-C₁₂ alkylamine;
subjecting the adduct to a MS technique to determine the amount of the adduct; and
calculating the amount of the analyte equivalent to the amount of the adduct,
wherein the method does not include a derivatisation step.

2. A method as claimed in claim 1 , wherein the vitamin D metabolite comprises one or more compounds selected from the group consisting of:
1-alpha-hydroxyvitamin D2, 1-alpha-hydroxyvitamin D3, 25-hydroxyvitamin D2, 25-hydroxyvitamin D3, 1,25-dihydroxyvitamin D2, 1,25-dihydroxyvitamin D3, (R) 24, 25 - dihydroxyvitamin D2, (R)24,25 dihydroxyvitamin D3,(S) 24,25-dihydroxyvitamin D2, (S)24,25 dihydroxyvitamin D3, 1,24,25-trihydroxyvitamin D2, and 1,24,25-trihydroxyvitamin D3.

3. A method as in claim 1 or 2, wherein the alkylamine is a C4-C8 alkylamine.

4. A method as in claim 1 or 2, wherein the alkylamine is a C6-C8 alkylamine.

5. A method as claimed in claim 1 or claim 2, wherein the amine is a diamine of formula H₂N(CH₂)ₙNH₂ wherein n is an integer from 4-12.

6. A method as claimed in any preceding claim, wherein the MS technique comprises a tandem (MS/MS) technique.

7. A method as claimed in claim 6, wherein the MS technique is a LC-MS/MS technique.

8. A method as claimed in any preceding claim, wherein the MS technique comprises a triple quadruple technique using Multiple Reaction Monitoring (MRM) in positive ion mode.

9. A method as claimed in claim 7, wherein the LC-MS/MS technique comprises a Q1 scan to detect a precursor corresponding to the analyte-amine adduct.

## Patentansprüche

1. Verfahren zur Bestimmung der Menge eines Analyts in einer Probe, worin der Analyt einen oder mehrere Vitamin D-Metabolite umfasst;
umfassend die folgenden Schritte:
Inkontaktbringen des Analyts mit einem Alkylamin, um ein Alkylaminaddukt zu bilden, worin das Amin ein Monoamin oder Diamin ist und worin das Alkylamin ein C₄-C₁₂-Alkylamin ist;
Unterziehen des Addukts einer MS-Technik, um die Menge des Addukts zu bestimmen; und
Berechnen der Menge des Analyts, die äquivalent zur Menge des Addukts ist,
worin das Verfahren keinen Derivatisierungsschritt beinhaltet.

2. Verfahren nach Anspruch 1, worin der Vitamin D-Metabolit eine oder mehrere Verbindungen umfasst, die ausgewählt sind aus der Gruppe bestehend aus:
1-Alpha-Hydroxyvitamin D2, 1-Alpha-Hydroxyvitamin D3, 25-Hydroxyvitamin D2, 25-Hydroxyvitamin D3, 1,25-Dihydroxyvitamin D2, 1,25-Dihydroxyvitamin D3, (R)24,25-Dihydroxyvitamin D2, (R)24,25-Dihydroxyvitamin D3, (S)24,25-Dihydroxyvitamin D2, (S)24,25-Dihydroxyvitamin D3, 1,24,25-Trihydroxyvitamin D2 und 1,24,25-Trihydroxyvitamin D3.

3. Verfahren nach Anspruch 1 oder 2, worin das Alkylamin ein C4-C8-Alkylamin ist.

4. Verfahren nach Anspruch 1 oder 2, worin das Alkylamin ein C6-C8-Alkylamin ist.

5. Verfahren nach Anspruch 1 oder Anspruch 2, worin das Amin ein Diamin der Formel H₂N(CH₂)ₙNH₂ ist, worin n eine ganze Zahl von 4-12 ist.

6. Verfahren nach einem vorhergehenden Anspruch, worin die MS-Technik eine Tandem- (MS/MS-) Technik umfasst.

7. Verfahren nach Anspruch 6, worin die MS-Technik eine LC-MS/MS-Technik ist.

8. Verfahren nach einem vorhergehenden Anspruch, worin die MS-Technik eine Dreifach-Vierfach-Technik unter Verwendung von Multiple Reaction Monitoring (MRM) im positiven Ionenmodus umfasst.

9. Verfahren nach Anspruch 7, worin die LC-MS/MS-Technik einen Q1-Scan zum Detektieren eines dem Analyt-Amin-Addukt entsprechenden Vorläufers umfasst.

## Revendications

1. Procédé de détermination de la quantité d'un analyte dans un échantillon, l'analyte comprenant un ou plusieurs métabolites de la vitamine D ;
ledit procédé comprenant les étapes consistant à :
mettre en contact l'analyte avec une alkylamine pour former un adduit d'alkylamine, l'amine étant une monoamine ou une diamine, et l'alkylamine étant une alkylamine en C4 à C12 ;
soumettre l'adduit à une technique de SM pour déterminer la quantité de l'adduit ; et
calculer la quantité de l'analyte équivalente à la quantité de l'adduit ;
le procédé ne comprenant pas d'étape de dérivation.

2. Procédé selon la revendication 1, dans lequel le métabolite de la vitamine D comprend un ou plusieurs composés choisis dans le groupe constitué de :
1-alpha-hydroxyvitamine D2, 1-alpha-hydroxyvitamine D3, 25-hydroxyvitamine D2, 25-hydroxyvitamine D3, 1,25-dihydroxyvitamine D2, 1,25-dihydroxyvitamine D3, (R)24,25-dihydroxyvitamine D2, (R)24,25-dihydroxyvitamine D3, (S)24,25-dihydroxyvitamine D2, (S)24,25-dihydroxyvitamine D3, 1,24,25-trihydroxyvitamine D2 et 1,24,25-trihydroxyvitamine D3.

3. Procédé selon la revendication 1 ou 2, dans lequel l'alkylamine est une alkylamine en C4 à C8.

4. Procédé selon la revendication 1 ou 2, dans lequel l'alkylamine est une alkylamine en C6 à C8.

5. Procédé selon la revendication 1 ou 2, dans lequel l'amine est une diamine de formule H2N(CH2)nNH2, où n est un entier de 4 à 12.

6. Procédé selon l'une quelconque des revendications précédentes, dans lequel la technique de SM consiste en une technique tandem (SM/SM).

7. Procédé selon la revendication 6, dans lequel la technique de SM est une technique de CL-SM/SM.

8. Procédé selon l'une quelconque des revendications précédentes, dans lequel la technique de SM comprend une technique de triple quadripôle au moyen d'un suivi de réactions multiples (MRM) en mode d'ions positifs.

9. Procédé selon la revendication 7, dans lequel la technique de CL-SM/SM comprend une analyse Q1 pour détecter un précurseur correspondant à l'analyte-adduit d'amine.
